(19) **Europäisches Patentamt / European Patent Office / Office européen des brevets**

(11) **EP 2 095 812 A1**

(12) **EUROPEAN PATENT APPLICATION**
published in accordance with Art. 153(4) EPC

(43) Date of publication:
**02.09.2009 Bulletin 2009/36**

(21) Application number: **07830588.5**

(22) Date of filing: **25.10.2007**

(51) Int Cl.:
*A61K 9/14* (2006.01)  *A61K 47/10* (2006.01)
*A61K 47/12* (2006.01)  *A61K 47/26* (2006.01)
*A61K 47/36* (2006.01)

(86) International application number:
**PCT/JP2007/070854**

(87) International publication number:
**WO 2008/050847 (02.05.2008 Gazette 2008/18)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **25.10.2006 JP 2006290561**

(71) Applicant: **Dainippon Sumitomo Pharma Co., Ltd. Osaka 541-8524 (JP)**

(72) Inventors:
• **MATSUI, Yasuhiro**
  **Ibaraki-shi**
  **Osaka 5670878 (JP)**
• **IKEDA, Yuki**
  **Ibaraki-shi**
  **Osaka 5670878 (JP)**
• **OCHIAI, Yasushi**
  **Ibaraki-shi**
  **Osaka 5670878 (JP)**

(74) Representative: **Duckworth, Timothy John**
  **J.A. Kemp & Co.,**
  **14 South Square,**
  **Gray's Inn**
  **London WC1R 5JJ (GB)**

(54) **GRANULAR PREPARATION PREVENTED FROM CAKING**

(57) The present invention provides a granular preparation that resists caking during preservation, namely, a granular preparation characteristically with suppressed caking, which comprises an active ingredient other than biguanide, a sugar or a sugar alcohol, an organic acid and a particular water-soluble polysaccharide; and a method of suppressing caking of a granular preparation, which includes adding a particular water-soluble polysaccharide to the active ingredient, sugar or sugar alcohol and organic acid.

EP 2 095 812 A1

**Description**

Technical Field

The present invention relates to a granular preparation comprising an active ingredient other than biguanide, a sugar or a sugar alcohol, an organic acid and a particular water-soluble polysaccharide, which resists easy formation of caking during preservation. The present invention also relates to a method of suppressing caking of a granular preparation, which comprises adding a particular water-soluble polysaccharide to a composition comprising an active ingredient other than biguanide, a sugar or a sugar alcohol, and an organic acid, and a caking suppressing agent.

Background Art

For pharmaceutical products, the effectiveness and safety of the medicinal component itself are important, as well as the stability of the medicinal component in a preparation and properties thereof from the aspects of formulation of preparation are also extremely important. For example, granular preparation is a dosage form often used in the medical practice, since it is easy to take, permits easy control of the dose, and shows good chemical stability from being solid. Even when a granular preparation satisfies a certain level of quality immediately after production, it sometimes forms caking during preservation depending on the properties of the medicinal component and additives therein. In such cases, the preparation may cause problems in terms of easy administration and handling of a pharmaceutical product.

For example, an orally dissolving solid preparation, which easily dissolves in the mouth cavity, can be taken without water, and is superior in cold and refreshing feeling, as achieved by a combined use of erythritol and a solid organic acid providing an acid taste, is disclosed (patent document 1). However, patent document 1 does not disclose the problem found by the present inventors relating to the caking in the above-mentioned preparation, which is caused by the addition of sugar alcohol and organic acid, much less a means of solving the problem.

As a method of suppressing caking of powder preparations, for example, it is known that a powder preparation for compromised skin repair, comprising a blend of white soft sugar, povidone iodine and a certain kind of water-soluble polymer carrier, is free of caking even after a long-term preservation (patent document 2). Although patent document 2 provides a description relating to the prevention of caking caused by white soft sugar, it is silent on a caking preventive effect provided by the addition of organic acid to sugar or sugar alcohol. Moreover, it does not specifically disclose a preparation with suppressed caking, which comprises pullulan and/or dextrin, the characteristic elements of the present invention.

    patent document 1: JP-A-9-316006
    patent document 2: JP-A-8-12582

Disclosure of the Invention

Problems to be Solved by the Invention

The present inventors have found a problem that addition of a sugar or a sugar alcohol and an organic acid to a granular preparation in order to improve dissolution property in the mouth cavity and afford superior administrability may lead to caking of the granular preparation during preservation. In this event, it is predictable that easy administration and easy handlability will be unattainable (difficulty when in use). Particularly, caking phenomenon is significant when the amount of the organic acid added to a granular preparation becomes high.

Means of Solving the Problems

Thus, the present inventors have conducted intensive studies in an attempt to suppress caking of a granular preparation comprising sugar or sugar alcohol and organic acid, and found that a preparation with suppressed caking during preservation can be unexpectedly obtained by adding at least one water-soluble polysaccharide selected from the group consisting of pullulan and dextrin to the preparation, which resulted in the completion of the present invention. Accordingly, the present invention provides the following.

"[1] A granular preparation with suppressed caking, which comprises an active ingredient other than biguanide, a sugar or a sugar alcohol, an organic acid, and at least one water-soluble polysaccharide selected from the group consisting of pullulan and dextrin.
[2] The granular preparation of [1], wherein the sugar or sugar alcohol is at least one selected from the group consisting of erythritol, mannitol, xylitol, sorbitol, maltitol and trehalose.

[3] The granular preparation of [1], wherein the sugar or sugar alcohol is at least one selected from the group consisting of erythritol and mannitol.

[4] The granular preparation of any one of [1] to [3], wherein the organic acid is at least one selected from the group consisting of citric acid, malic acid, ascorbic acid, tartaric acid, succinic acid and hydrates thereof.

[5] The granular preparation of any one of [1] to [3], wherein the organic acid is at least one selected from the group consisting of citric acid, malic acid and hydrates thereof.

[6] The granular preparation of any one of [1] to [5], wherein the water-soluble polysaccharide is pullulan.

[7] The granular preparation of any one of [1] to [6], wherein the proportion of sugar or sugar alcohol is 20 - 99.8 wt% of the whole preparation.

[8] The granular preparation of any one of [1] to [7], wherein the proportion of the organic acid is 0.1 - 30 wt% of the whole preparation.

[9] The granular preparation of any one of [1] to [8], wherein the proportion of the water-soluble polysaccharide is 0.1 - 20 wt% of the whole preparation.

[10] The granular preparation of any one of [1] to [9], wherein the weight ratio of an addition amount of the water-soluble polysaccharide to an addition amount of the organic acid is 0.01 - 100.

[11] The granular preparation of any one of [1] to [10], which has a 90% diameter of not more than 1700 $\mu$m.

[12] The granular preparation of any one of [1] to [11], wherein the dosage form is an orally-disintegrating preparation.

[13] A method of suppressing caking of a granular preparation, which comprises a step of adding at least one water-soluble polysaccharide selected from the group consisting of pullulan and dextrin to a preparation comprising an active ingredient other than biguanide, a sugar or a sugar alcohol and an organic acid.

[14] A method of producing a granular preparation with suppressed caking, which comprises a step of adding at least one water-soluble polysaccharide selected from the group consisting of pullulan and dextrin to an active ingredient other than biguanide, a sugar or sugar alcohol, and an organic acid.

[15] A caking suppressing agent comprising at least one water-soluble polysaccharide selected from the group consisting of pullulan and dextrin, which agent is used for a granular preparation comprising an active ingredient other than biguanide, a sugar or a sugar alcohol and an organic acid.

[16] A granular preparation with suppressed caking, which comprises a sugar or a sugar alcohol, an organic acid, and at least one water-soluble polysaccharide selected from the group consisting of pullulan and dextrin, and does not comprise biguanide."

Effect of the Invention

The granular preparation of the present invention provides effects in that it resists caking during preservation, and permits easy administration, and is useful as a preparation such as a pharmaceutical product and the like.

Best Mode for Carrying out the Invention

The active ingredient in the present invention may be any as long as it is other than biguanide. Here, biguanide means drugs having a biguanide skeleton, and encompasses those in the form of pharmacologically acceptable salts such as hydrochloride and the like. Examples thereof include metformin, buformin, phenformin and the like, and pharmacologically acceptable salts thereof. The active ingredient may be a natural one derived from animal or plant, or one obtained by a chemical synthesis method or fermentation method. In addition, salts thereof are also encompassed in the active ingredient of the present invention. In the present invention, the active ingredient may be in the form of a solid, a liquid, an oil and the like. When the medicinal component in the present invention is a solid, it may be a crystal or amorphous.

Examples of the active ingredient in the present invention include nutritional supplements; antipyretic/antiphlogistic analgetics; antipsychotic drugs; hypnotic sedatives; antispasmodic drugs; central nervous system drugs; cerebral metabolism-improving drugs; cerebral circulation-improving drugs; antiepileptic drugs; sympathomimetic agents; stomachics and digestives; antiulcerogenic drugs; gastroprokinetics; antacids; anti-tussive expectorants; antimotility agents; antiemetics; respiratory stimulants; bronchodilators; antiallergic drugs; anti-histamines; cardiotonics; antiarrhythmic agents; diuretics; ACE inhibitors; Ca antagonists; AII antagonists; vasoconstrictors; coronary vasodilators; vasodilators; peripheral vasodilators; antihyperlipidemia agents; cholagogues; cephem antibiotics; oral antibacterial agents; chemical therapeutic agent; sulfonylurea drug; $\alpha$ glucosidase inhibitor; insulin sensitizer; rapid insulin secretagogue; DPPIV inhibitor; therapeutic drug for diabetic complications; anti-osteoporosis drug; antirheumatic drug; skeletal muscle relaxant; anti-motionsickness agents; narcotic alkaloids; sulfa drugs; gout therapeutic agents; anticoagulants; anticancer drugs and the like.

Specifically, the active ingredient in the present invention includes nutritional supplements such as vitamins, minerals, amino acid, crude drug, lactobacillus and the like; antipyretic/antiphlogistic analgetics such as aspirin, aceta-

# EP 2 095 812 A1

minophen, ethenzamide, ibuprofen, caffeine, indomethacin and the like; antipsychotic drugs such as blonanserin, lurasidone (or lurasidone hydrochloride), tandospirone citrate, perospirone hydrochloride, reserpine, diazepam, fludiazepam, haloperidol, aripiprazole, nortriptyline hydrochloride and the like; hypnotic sedatives such as nitrazepam, diazepam, triazolam, brotizolam, zolpidem, nimetazepam and the like; antispasmodic drugs such as scopolamine hydrobromide and the like; central nervous system drugs such as zonisamide, droxidopa, citicoline, biperiden hydrochloride, donepezil hydrochloride, 5-(3-methoxyphenyl)-3-(5-methyl-1,2,4-oxadiazol-3-yl)-1,6-naphthyridin-2(1H)-one and the like; cerebral metabolism-improving drugs such as meclofenoxate hydrochloride and the like; cerebral circulation-improving drugs such as vinpocetine and the like; antiepileptic drugs such as zonisamide, phenytoin, clonazepam, primidone, sodium valproate, carbamazepine, diazepam, ethotoin, acetylpheneturide and the like; sympathomimetic agents such as isoproterenol hydrochloride and the like; stomachics and digestives such as diastase, scopolia extract, pancreatin and the like; antiulcerogenic drugs such as cimetidine, lansoprazole, famotidine, sulpiride, gefarnate and the like; gastroprokinetics such as mosapride citrate and the like; antacids such as magnesium alumino metasilicate and the like; anti-tussive expectorants such as cloperastine hydrochloride, ephedrine hydrochloride, pentoxyverine citrate and the like; antimotility agents such as loperamide hydrochloride and the like; antiemetics such as difenidol hydrochloride and the like; respiratory stimulants such as levallorphan tartrate and the like; bronchodilators such as theophylline and the like; antiallergic drugs such as ebastine, N-{2-[2-[(3-fluorophenyl)imino]-4-(4-morpholin-4-ylphenyl)-1,3-thiazol-3(2H)-yl]ethyl}-N'-methylurea and the like; anti-histamines such as diphenhydramine hydrochloride and the like; cardiotonics such as caffeine, digoxin and the like; antiarrhythmic agents such as procaineamide hydrochloride, arotinolol hydrochloride and the like; diuretics such as isosorbide and the like; ACE inhibitors such as delapril hydrochloride, captopril, alacepril and the like; Ca antagonists such as nifedipine, diltiazem hydrochloride, manidipine hydrochloride, amlodipine besylate and the like; AII antagonists such as candesartan, irbesartan, olmesartan, valsartan and the like; vasoconstrictors such as phenylephrine hydrochloride and the like; coronary vasodilators such as carbochromene hydrochloride and the like; vasodilators such as limaprostalfadex and the like; peripheral vasodilators such as cinnarizinee and the like; antihyperlipidemia agents such as simvastatin, pravastatin sodium and the like; cholagogues such as dehydrocholic acid and the like; cephem antibiotics such as cephalexin, cefaclor and the like; oral antibacterial agents such as gatifloxacin, sparfloxacin and the like; chemical therapeutic agents such as sufamethizol, pipemidic acid trihydrate and the like; sulfonylurea drugs such as gliclazide, glibenclamide, glimepiride and the like; $\alpha$ glucosidase inhibitors such as acarbose, voglibose, miglitol and the like; insulin sensitizers such as pioglitazone hydrochloride, rosiglitazone and the like; rapid insulin secretagogues such as nateglinide, mitiglinide calcium hydrate, repaglinide and the like; DPPIV inhibitors such as itagliptin and the like; therapeutic drug for diabetic complications such as ranirestat, epalrestat and the like; antiosteoporosis drugs such as etidronate disodium and the like; antirheumatic drugs such as methotrexate and the like; skeletal muscle relaxants such as methocarbamol and the like; anti-motionsickness agents such as meclizine hydrochloride and the like; narcotic alkaloids such as morphine hydrochloride, opium and the like; sulfa drugs such as sulfisomidine and the like; gout therapeutic agents such as allopurinol and the like; anticoagulants such as dicoumarol and the like; anticancer drugs such as 5-fluorouracil, mitomycin and the like; and the like.

More specifically, antipsychotic drugs, central nervous system drug, gastroprokinetics, antiallergic drug, sulfonylurea drug , therapeutic drug for diabetic complications, and the like exemplified above can be mentioned.

The active ingredient in the present invention may be selected from indomethacin, blonanserin, lurasidone (or lurasidone hydrochloride), tandospirone citrate, perospirone hydrochloride, fludiazepam, haloperidol, nortriptyline hydrochloride, nimetazepam, zonisamide, 5-(3-methoxyphenyl)-3-(5-methyl-1,2,4-oxadiazol-3-yl)-1,6-naphthyridine-2(1H)-one, N-{2-[2-[(3-fluorophenyl)imino]-4-(4-morpholin-4-ylphenyl)-1,3-thiazole-3(2H)-yl]ethyl}-N'-methylurea, droxidopa, biperiden hydrochloride, phenytoin, clonazepam, primidone, sodium valproate, ethotoin, acetylpheneturide, pancreatin, cimetidine, sulpiride, gefarnate, mosapride citrate, ephedrine hydrochloride, pentoxyverine citrate, arotinolol hydrochloride, alacepril, amlodipine besylate, gatifloxacin, sparfloxacin, pipemidic acid trihydrate, gliclazide, miglitol, repaglinide, ranirestat, etidronate disodium , allopurinol and the like.

More specifically, blonanserin, 5-(3-methoxyphenyl)-3-(5-methyl-1,2,4-oxadiazol-3-yl)-1,6-naphthyridin-2(1H)-one, N-{2-[2-[(3-fluorophenyl)imino]-4-(4-morpholin-4-ylphenyl)-1,3-thiazol-3(2H)-yl]ethyl}-N'-methylurea, perospirone hydrochloride, lurasidone (or lurasidone hydrochloride), zonisamide, mosapride citrate, gliclazide, ranirestat and the like can be mentioned.

The active ingredients recited above may be in the form of a salt or a free form other than those indicated above, as long as it is pharmacologically acceptable. In addition, they may be in the form of a solvate such as alcohol hydrate and the like, or a hydrate and the like. Moreover, the active ingredients recited above may be used alone or two or more kinds thereof may be combined. In addition, they may be subjected to a treatment to mask an uncomfortable taste such as a bitterness of the active ingredient and the like.

While the content of the active ingredient in the present invention varies depending on the component thereof, it is generally not less than 0.001 wt%, preferably not less than 0.01 wt%, more preferably not less than 0.05 wt, and generally not more than 30 wt%, preferably not more than 20 wt%, more preferably not more than 10 wt%, relative to the amount of the granular preparation excluding water-soluble polysaccharide(s) (pullulan and/or dextrin). More specifically, for example, it is 0.001 - 30 wt%, preferably 0.01 - 20 wt%, more preferably 0.05 - 10 wt%.

The sugar or sugar alcohol in the present invention may be a natural one derived from animal or plant, or one obtained by a chemical synthesis method or fermentation method. Here, examples of sugar include glucose, fructose, trehalose, palatinose and the like. Glucose, trehalose and palatinose are preferable, and among these, trehalose is more preferable. Examples of sugar alcohol include erythritol, mannitol, xylitol, sorbitol, maltitol and the like. Among these, erythritol, mannitol, xylitol, sorbitol and multitol are preferable, and erythritol and mannitol are more preferable. These sugar and sugar alcohol may be used alone, or two or more kinds thereof may be used in combination according to a desired preparation.

Examples of the organic acid in the present invention include citric acid, malic acid, ascorbic acid, tartaric acid, succinic acid, adipic acid, fumaric acid, maleic acid, gluconic acid, glucuronic acid and hydrates thereof and the like. Among these, citric acid, malic acid, ascorbic acid, tartaric acid, succinic acid and hydrates thereof are preferable. Particularly, citric acid, malic acid and hydrates thereof are more preferable. These organic acids may be used alone or two or more kinds thereof may be used in combination according to a desired preparation.

As the caking suppressing agent in the present invention, pullulan and dextrin are preferable from among the water-soluble polymers and water-soluble polysaccharides, since they provide a high caking suppressive effect. Pullulan and dextrin may be used alone or two or more kinds thereof may be used in combination according to a desired preparation. Particularly, pullulan is preferable.

Pullulan in the present invention is a natural polysaccharide, wherein maltotriose is regularly $\alpha$-1,6 bonded, which is generally obtained by culturing *Aureobasidium pullulans*, one kind of black yeast, using starch as a starting material. Preferably, one free of modification (e.g., introduction of substituent by a chemical reaction and the like) can be employed. Pullulan is not particularly limited as long as its use as a pharmaceutical product is acceptable. The average molecular weight is preferably 10,000 - 1,000,000, more preferably 50,000 - 500,000, more preferably 100,000 - 300,000.

Dextrin in the present invention is a generic term of polysaccharides represented by the formula $(C_6H_{10}O_5)_n \cdot x$ $H_2O$, which are intermediate products generally obtained by a heat treatment of dry starch, before reaching maltose. Dextrin is not particularly limited as long as its use as a pharmaceutical product is acceptable. The average molecular weight is preferably 1000 - 20000, more preferably 2000 - 10000, still more preferably 3000 - 6000.

The granular preparation of the present invention comprises a sugar or a sugar alcohol in a proportion of 20 - 99.8 wt%, preferably 20 - 99 wt%, more preferably 50 - 99 wt%, still more preferably 75 - 98 wt%, of the preparation, from the aspects of suppression of caking, easy administration and the like. The organic acid is contained in a proportion of 0.1 - 30 wt%, preferably 0.5 - 20 wt%, more preferably 1 - 10 wt%, of the preparation. The water-soluble polysaccharide (s) (pullulan and/or dextrin) is contained in a proportion of 0.1 - 20 wt%, preferably 0.5 - 15 wt%, more preferably 1 - 10 wt%, of the preparation.

In the granular preparation of the present invention, the weight ratio of an addition amount of water-soluble polysaccharide(s) (pullulan and/or dextrin) and an addition amount of organic acid (addition amount of water-soluble polysaccharide/addition amount of organic acid) is generally not less than 0.01, preferably not less than 0.02, more preferably not less than 0.1, particularly preferably not less than 0.65, from the aspect of suppression of caking. While the upper limit is not particularly limited, it is generally not more than 100, preferably not more than 50, more preferably not more than 25, and particularly preferably not more than 15.

The granular preparation in the present invention is preferably in the form of granules having a particle size of 1700 $\mu$m or below. Unless otherwise specified, the particle size in the present specification means a 90% diameter, which is a particle size at the point of intersection between the distribution curve of integrated % of particles (based on volume) and 90% on the horizontal axis. The particle size is measured, for example, by a dry measurement (injection type) using a laser diffraction particle size distribution measurement apparatus (SALD-3000 SHIMADZU Corporation).

The particle size (90% diameter) of the granular preparation in the present invention is generally not more than 1700 $\mu$m, preferably not more than 850 $\mu$m, more preferably not more than 500 $\mu$m, particularly preferably not more than 350 $\mu$m, since good administrability can be obtained. It is also preferable that a 10% diameter (a 10% diameter, which is a particle size at the point of intersection between the distribution curve of integrated % of particles (based on volume) and 10% on the horizontal axis) be not less than 75 $\mu$m, since good administrability can be obtained.

The orally-disintegrating preparation in the present invention refers to a preparation in which the entire amount taken into the mouth cavity is dissolved or disintegrated to 200 $\mu$m or below within 30 sec. When the entire amount taken is dissolved or disintegrated to 75 $\mu$m or below within 15 sec, more preferable administrability is obtained.

As mentioned above, the granular preparation of the present invention obtained by blending the active ingredient with a sugar or a sugar alcohol, an organic acid, and at least one water-soluble polysaccharide selected from the group consisting of pullulan and dextrin is a useful preparation, since it is superior in handling since caking during preservation is suppressed, shows improved dissolution property in the mouth cavity as compared to the use of sugar or sugar alcohol alone, which is attributable to the addition of an organic acid, and affords superior administrability by the addition of an acid taste.

In the present invention, "caking" means strong coagulation of a granular preparation, which cannot be easily restored to the original state with a weak physical stimulation, and "resist caking" means a state which can be easily restored to the original state with a weak physical stimulation or complete absence of coagulation.

For example, the level of caking can be evaluated by determining wt% of each of good, coagulation (weak), coagulation (medium) and coagulation (strong) in a granular preparation using the evaluation method described in the Examples of the present specification, and evaluating the total of coagulation (medium) and coagulation (strong) as "wt% of granule caking". In the present invention, "suppressing caking" can be judged by a decrease in "wt% of granule caking" after preservation under particular preservation conditions as compared to that of a preparation without a caking suppressing agent. As long as a decrease is observed, the level of decrease is not questioned. For example, the "wt% of granule caking" of a preparation comprising a caking suppressing agent after preservation at 40°C for 4 days is 0.9-fold or below, preferably 0.7-fold or below, more preferably 0.5-fold or below, and still more preferably 0.25-fold or below, based on the "wt% of granule caking" after preservation of a preparation without a caking suppressing agent under the same conditions.

A caking suppressing agent in the present invention is added to suppress caking of a granular preparation comprising an active ingredient other than biguanide, a sugar or a sugar alcohol and an organic acid during preservation, and is an agent comprising at least one water-soluble polysaccharide selected from the group consisting of pullulan and dextrin.

The preferable active ingredient, sugar or sugar alcohol, organic acid, water-soluble polysaccharide(s) (pullulan and/or dextrin) and addition amounts thereof and the like are as mentioned above. When the above-mentioned pullulan and dextrin are used as caking suppressing agents, superior effects of quick dissolution in the mouth cavity, no influence on the taste and easy administration are particularly afforded.

The granular preparation of the present invention can be produced by a granulation method known per se. For example, the preparation can be produced by blending the above-mentioned active ingredient with a sugar or a sugar alcohol, and an organic acid by a blending method generally employed for preparations, granulating and drying the mixture, and adding water-soluble polysaccharide(s) (pullulan and/or dextrin). Examples of the granulation method include extrusion-granulation method, fluidized bed granulation method, rotor granulation method, agitation granulation method and the like. In view of productivity, extrusion-granulation method, fluidized bed granulation method and agitation granulation method are preferable, and fluidized bed granulation method is more preferable.

In addition, a method including blending the above-mentioned active ingredient with a sugar or a sugar alcohol, an organic acid and water-soluble polysaccharide(s) (pullulan and/or dextrin) by a blending method generally employed for preparations, granulating and drying the mixture in a conventional manner to give a granular preparation; a method including spraying a solution of water-soluble polysaccharide(s) (pullulan and/or dextrin) on a granular preparation comprising an active ingredient, a sugar or a sugar alcohol and an organic acid by a known production method; a method including granulating an active ingredient, a sugar or a sugar alcohol and an organic acid while spraying a solution of water-soluble polysaccharide(s) (pullulan and/or dextrin) thereon to give a granular preparation and the like are available. Using the granular preparation obtained by the present invention and by a known press molding method, a tablet comprising the above-mentioned active ingredient can be produced.

In the present invention, the granular preparation may comprise nontoxic and inactive additive generally employed in the pharmaceutical field. Examples of such additive include those substantially uninfluential on the effect of the invention and generally used for oral preparations.

For example, binders such as gum arabic, gelatin, methylcellulose, polyvinylpyrrolidone, hydroxypropylcellulose (hereinafter sometimes to be abbreviated as HPC), hydroxypropylmethylcellulose, cornstarch and the like, high intensity sweeteners such as dipotassium glycyrrhizate, saccharin, sodium saccharin, stevia, thaumatin, neotame, aspartame, acesulfame potassium, sucralose and the like, colorants, corrigents, flavors/essences and the like may be added. It is also possible to add flavors such as lemon, lemonlime, grape, Japanese apricot, yogurt and the like, and the like. In this case, more preferable administrability can be afforded.

Moreover, since some of the active ingredients used for the granular preparation of the present invention have an uncomfortable taste such as bitterness and the like, the active ingredient may be subjected to a treatment to mask an uncomfortable taste before use. Such an uncomfortable taste can be masked by a coating method known per se. As the coating agent, those generally employed such as hydroxypropylmethylcellulose, hydroxypropylcellulose, methylcellulose, polyvinylpyrrolidone and the like can be used. As a coating aid, polyethylene glycol 6000, polysorbate (e.g., Tween 80 and the like), talc, titanium oxide, dye and the like can be used.

The granular preparation of the present invention can be safely used as a pharmaceutical agent or food (including functional food and the like) for mammals (e.g., human, dog, rabbit, rat, mouse and the like).

The method of suppressing caking in the present invention is a method of suppressing caking of a granular preparation by adding at least one water-soluble polysaccharide selected from the group consisting of pullulan and dextrin to a preparation comprising an the active ingredient other than biguanide, a sugar or a sugar alcohol and an organic acid. Examples thereof include methods of suppressing caking such as a method including blending water-soluble polysaccharide(s) (pullulan and/or dextrin) with the above-mentioned granular preparation comprising an active ingredient, a sugar or a sugar alcohol and an organic acid; a method including praying a solution of a water-soluble polysaccharide on the above-mentioned granular preparation comprising an active ingredient, a sugar or a sugar alcohol and an organic acid by a known production method; a method including granulating the above-mentioned active ingredient, a sugar or a sugar alcohol and an organic acid while spraying a solution of a water-soluble polysaccharide thereon; and a method including mixing the above-mentioned active ingredient, a sugar or a sugar alcohol, an organic acid and a water-soluble polysaccharide and granulating the mixture by a known method. A preferable method is a method including blending water-soluble polysaccharide(s) (pullulan and/or dextrin) with the above-mentioned granular preparation comprising an active ingredient, a sugar or a sugar alcohol and an organic acid (more specifically, a method including blending a water-soluble polysaccharide in a powder state). Preferable active ingredient, a sugar or a sugar alcohol, an organic acid, water-soluble polysaccharide(s) (pullulan and/or dextrin) and addition amounts thereof and the like are as mentioned above.

The production method of a granular preparation with suppressed caking in the present invention is a production method characteristically including adding at least one water-soluble polysaccharide selected from the group consisting of pullulan and dextrin to an active ingredient other than biguanide, a sugar or a sugar alcohol and an organic acid. Examples thereof include a production method including a step of preparing the above-mentioned granular preparation comprising an active ingredient, a sugar or a sugar alcohol and an organic acid and a step of adding water-soluble polysaccharide(s) (pullulan and/or dextrin); a production method including blending the above-mentioned active ingredient with a sugar or a sugar alcohol, an organic acid and water-soluble polysaccharide(s) (pullulan and/or dextrin) by a blending method generally used for preparations, and preparing a granular preparation by a known method; a production method including a step of preparing the above-mentioned granular preparation comprising an active ingredient, a sugar or a sugar alcohol, and an organic acid and a step of spraying a solution of water-soluble polysaccharide(s) (pullulan and/or dextrin) on the granular preparation; a production method including preparing a granular preparation by spraying a solution water-soluble polysaccharide(s) (pullulan and/or dextrin) on the above-mentioned active ingredient, a sugar or a sugar alcohol, and an organic acid and the like. Among these, a preferable production method is a production method including a step of preparing the above-mentioned granular preparation comprising an active ingredient, a sugar or a sugar alcohol and an organic acid and a step of adding water-soluble polysaccharide(s) (pullulan and/or dextrin). Preferable examples of the active ingredient, sugar or sugar alcohol, organic acid, water-soluble polysaccharide(s) (pullulan and/or dextrin) and addition amounts thereof and the like are as mentioned above.

Another embodiment of the present invention is a granular preparation with suppressed caking, which characteristically comprises a sugar or a sugar alcohol, an organic acid, and at least one water-soluble polysaccharide selected from the group consisting of pullulan and dextrin, but does not comprise biguanide. The granular preparation can be used as a pharmaceutical agent, an additive for pharmaceutical products and the like. Preferable examples of the sugar or sugar alcohol, organic acid, water-soluble polysaccharide(s) (pullulan and/or dextrin) and addition amounts thereof and the like are as mentioned above.

## Examples

The present invention is explained in detail in the following by referring to Examples, which are not to be construed as limitative.

Unless particularly indicated, the following compounds and reagents were used in the following Examples, Comparative Examples and Experimental Examples. pullulan: PI-20 finely milled powder, HAYASHIBARA SHOJI. INC dextrin: Pinedex #2, Matsutani Chemical Industry Co., Ltd. compound (I): (3R)-2'-(4-bromo-2-fluorobenzyl)spiro[pyrrolidine-3,4'(1'H)-pyrrolo[1,2-a]pyrazine]-1',2,3',5(2'H)-tetraone (ranirestat; can be synthesized according to the method described in, for example, JP-B-2516147 and the like)

compound (II): N-{2-[2-[(3-fluorophenyl)imino]-4-(4-morpholin-4-ylphenyl)-1,3-thiazol-3(2H)-yl]ethyl}-N'-methylurea (can be synthesized according to the method described in, for example, WO2006/118268 and the like)

compound (III): 5-(3-methoxyphenyl)-3-(5-methyl-1,2,4-oxadiazol-3-yl)-1,6-naphthyridin-2(1H)-one (can be synthesized according to the method described in, for example, WO99/03857 and the like)

1. Caking enhancing effect of organic acid

(1) Extrusion granulation method

Erythritol (finely milled powder), mannitol or xylitol (finely milled powder, 22.5 g) and an organic acid (2.5 g) were measured and mixed in a mortar (amount of organic acid added 10%). As the organic acid, anhydrous citric acid (ground product) or malic acid was used. They were kneaded in a mortar for about 5 min while gradually adding water (0.4 g), and further kneaded in a mortar for about 5 min while gradually adding water (0.4 g). The kneaded product was dried at 40°C for about 15 min, and passed through a sieve with aperture of 500 μm. The product was dried at 40°C for about 1 - 2 hr, and passed through a sieve with aperture of 500 μm. In the same manner except addition of an organic acid, a preparation without an organic acid was obtained.

(2) Preservation method

From the obtained preparation, 4 g was placed in a white glass bottle (size: No. 2, manufactured by OSAKA GLASS CO., LTD.), and the bottle was tightly sealed and preserved at 40°C for 7 days. In the following Examples and the like, a preparation of Example (4.5 g) or a preparation of Comparative Example (4 g) was placed in the above-mentioned white glass bottle, which was tightly sealed and preserved at 40°C or 35°C for a given period.

(3) Evaluation method

1) The weight (A) of sieve (aperture 2000 μm) and weight (B) of sieve pan are measured.
2) The cap of the preservation container is removed, the container is gently turned over on the sieve, and the weight (D) of the sieve pan is measured.
3) When the preparation remains in the preservation container, the container is capped, turned over, and freely dropped 3 times from a 2 cm height to apply a drop impact.
4) The cap of the container is removed, and the container is gently turned over on the sieve.
5) The sieve is freely dropped 3 times from a 2 cm height to apply a drop impact, and the weight (E) of the sieve and the weight (F) of the sieve pan are measured.
6) When the preparation remains in the preservation container, the weight (G) of the preservation container containing the remaining preparation is measured.
7) The remaining preparation is removed from the preservation container, and the weight (H) of the preservation container itself is measured.
8) The total weight of the preparation discharged from the preservation container is determined (T=(D-B)+(F-D)+(E-A)+(G-H)).
9) The wt% of good, coagulation (weak), coagulation (medium) and coagulation (strong) is determined by the following formulas, and the sum of coagulation (medium) and coagulation (strong) is taken as "wt% of granule caking".

(calculation formulas)

$$good = (D-B)/T \times 100$$

$$coagulation\ (weak) = (F-D)/T \times 100$$

$$coagulation\ (medium) = (E-A)/T \times 100$$

$$coagulation\ (strong) = (G-H)/T \times 100$$

In the following Examples, Experimental Examples and the like, the level of caking was evaluated by the "wt% of granule caking" defined above.
The results are shown in Table 1. It was shown that addition of an organic acid enhanced caking.
Table 1

| | | wt% of granule caking | | |
|---|---|---|---|---|
| | | without organic acid | citric acid addition | malic acid addition |
| sugar alcohol | mannitol | 1 | 94 | 13 |
| | erythritol | 1 | 15 | 48 |
| | xylitol | 13 | 100 | 100 |

(4) Fluidized bed granulation method

Erythritol (finely milled powder, 800 g) and anhydrous citric acid (ground product, 100 g) were weighed (amount of organic acid added 11.1%), mixed in a polyethylene (hereinafter PE) bag with hands, and passed through a sieve with aperture of 500 $\mu$m. The sieved product was granulated in a fluidized bed granulator (POWREX CORPORATION Multiplex MP-01) while spraying 1% cornstarch solution [cornstarch (5 g) was dissolved in purified water (450 g) and the mixture was heated to 95°C, cooled to ambient temperature and purified water was added to the total weight of 500 g. A 1% cornstarch solution prepared in the same manner was also used in the following Examples and the like] at 6 g/min for 20 min and at 10 g/min for 38 min under the conditions of charge air temperature 70°C, air amount 70 m$^3$/hr, spray pressure 0.15 MPa. After granulation, the granules were dried for about 20 min under the conditions of charge air temperature 70°C, air amount 70 m$^3$/hr. A preparation comprising erythritol alone and free of anhydrous citric acid (erythritol charge amount 900 g) was also obtained in the same manner.
The preparation was preserved at 40°C for 7 days. The results are shown in Table 2. It was shown that addition of citric acid enhanced caking.
Table 2

| | wt% of granule caking | |
|---|---|---|
| | without organic acid | citric acid addition |
| erythritol | 0 | 15 |

2. Caking suppressive effect of water-soluble polysaccharide(s) (pullulan and/or dextrin)

(1) Extrusion granulation method

Comparative Example 1 (without addition of pullulan and dextrin)

Erythritol (finely milled powder) or mannitol and an organic acid were weighed as shown in Table 3 and mixed in a mortar (amount of organic acid added 10%). They were kneaded in a mortar for about 5 min while gradually adding water (0.4 g), and further kneaded in a mortar for about 5 min while gradually adding water (0.4 g). The kneaded product was dried at 40°C for about 15 min, and passed through a sieve with aperture of 500 $\mu$m. The product was dried at 40°C for about 1 - 2 hr, and passed through a sieve with aperture of 500 $\mu$m.
Table 3

| ingredient | amount added (g) | | | | | | | | | |
|---|---|---|---|---|---|---|---|---|---|---|
| erythritol | 22.5 | 22.5 | 22.5 | 22.5 | 22.5 | - | - | - | - | - |
| mannitol | - | - | - | - | - | 22.5 | 22.5 | 22.5 | 22.5 | 22.5 |
| anhydrous citric acid (ground product) | 2.5 | - | - | - | - | 2.5 | - | - | - | - |
| malic acid | - | 2.5 | - | - | - | - | 2.5 | - | - | - |
| ascorbic acid | - | - | 2.5 | - | - | - | - | 2.5 | - | - |
| tartaric acid | - | - | - | 2.5 | - | - | - | - | 2.5 | - |

(continued)

| ingredient | amount added (g) | | | | | | | | | |
|---|---|---|---|---|---|---|---|---|---|---|
| succinic acid | - | - | - | - | 2.5 | - | - | - | - | 2.5 |

Example 1 (with pullulan addition)

Pullulan (0.5 g) was added to the preparation (4 g) produced by the method shown in the above-mentioned Comparative Example 1, and they were mixed in a PE bag for about 5 min.

Example 2 (with dextrin addition)

Dextrin (0.5 g) was added to the preparation (4 g) produced by the method shown in the above-mentioned Comparative Example 1, and they were mixed in a PE bag for about 5 min.

Comparative Example 2 (without pullulan addition)

Xylitol (finely milled powder), sorbitol, maltitol (powder MABIT(R) 100M, HAYASHIBARA SHOJI. INC), fructose, trehalose, sucrose or palatinose and an organic acid were weighed as shown in Table 4 and Table 5 and mixed in a mortar (amount of organic acid added 10%). They were kneaded in a mortar for about 5 min while gradually adding water (0.4 g), and further kneaded in a mortar for about 5 min while gradually adding water (0.4 g). The kneaded product was dried at 40°C for about 15 min, and passed through a sieve with aperture of 500 $\mu$m. The product was dried at 40°C for about 1 - 2 hr, and passed through a sieve with aperture of 850 or 500 $\mu$m.

Table 4

| ingredient | amount added (g) | | | | | | | | | |
|---|---|---|---|---|---|---|---|---|---|---|
| xylitol | 22.5 | 22.5 | - | - | - | - | - | - | - | - |
| sorbitol | - | - | 22.5 | 22.5 | - | - | - | - | - | - |
| maltitol | - | - | - | - | 22.5 | 22.5 | - | - | - | - |
| fructose | - | - | - | - | - | - | 22.5 | 22.5 | - | - |
| trehalose | - | - | - | - | - | - | - | - | 22.5 | 22.5 |
| anhydrous citric acid (ground product) | 2.5 | - | 2.5 | - | 2.5 | - | 2.5 | - | 2.5 | - |
| malic acid | - | 2.5 | - | 2.5 | - | 2.5 | - | 2.5 | - | 2.5 |

Table 5

| ingredient | amount added (g) | | | |
|---|---|---|---|---|
| sucrose | 22.5 | 22.5 | - | - |
| palatinose | - | - | 22.5 | 22.5 |
| anhydrous citric acid (ground product) | 2.5 | - | 2.5 | - |
| malic acid | - | 2.5 | - | 2.5 |

Example 3 (with pullulan addition)

Pullulan (0.5 g) was added to the preparation (4 g) produced by the method shown in the above-mentioned Comparative Example 2, and they were mixed in a PE bag for about 5 min.

Comparative Example 3 (without pullulan addition)

Glucose or lactose and an organic acid were weighed as shown in Table 6 and mixed in a mortar (amount of organic acid added 30%). They were kneaded in a mortar for about 5 min while gradually adding water (0.64 g), and further kneaded in a mortar for about 5 min while gradually adding water (0.64 g). The kneaded product was dried at 40°C for about 15 min, and passed through a sieve with aperture of 500 μm. The product was dried at 40°C for about 1 - 2 hr, and passed through a sieve with aperture of 850 μm.

Table 6

|  | amount added (g) | | | |
|---|---|---|---|---|
| glucose | 14 | 14 | - | - |
| lactose | - | - | 14 | 14 |
| anhydrous citric acid (ground product) | 6 | - | 6 | - |
| malic acid | - | 6 | - | 6 |

Example 4 (with pullulan addition)

Pullulan (0.5 g) was added to the preparation (4 g) produced by the method shown in the above-mentioned Comparative Example 3, and they were mixed in a PE bag for about 5 min.

Experimental Example 1

The preparations of Comparative Examples 1 - 3 and Examples 1 - 4 were evaluated for the level of caking according to the preservation method and evaluation method described above.
The results are shown in Tables 7 and 8. It was shown that mixing of sugar or sugar alcohol and organic acid caused caking; on the other hand, pullulan, which is a water-soluble polysaccharide, showed a caking suppressive effect. Similarly, a caking suppressive effect was observed by dextrin, which is a water-soluble polysaccharide.

Table 7-1

| sugar or sugar alcohol | organic acid | organic acid (%) | preservation conditions | wt% of granule caking | |
|---|---|---|---|---|---|
|  |  |  |  | Comparative Example without pullulan | Example pullulan addition |
| erythritol | citric acid | 10 | 40°C×6 days | 15 | 0 |
|  | malic acid | 10 | 40°C×6 days | 48 | 6 |
|  | ascorbic acid | 10 | 40°C×6 days | 100 | 0 |
|  | tartaric acid | 10 | 40°C×6 days | 100 | 1 |
|  | succinic acid | 10 | 40°C×6 days | 32 | 0 |
| xylitol | citric acid | 10 | 40°C×6 days | 100 | 100 |
|  | malic acid | 10 | 40°C×6 days | 100 | 100 |
| mannitol | citric acid | 10 | 40°C×6 days | 94 | 0 |
|  | malic acid | 10 | 40°C×6 days | 13 | 0 |
|  | ascorbic acid | 10 | 40°C×6 days | 100 | 2 |
|  | tartaric acid | 10 | 40°C×6 days | 14 | 0 |
|  | succinic acid | 10 | 40°C×6 days | 100 | 18 |

(continued)

| sugar or sugar alcohol | organic acid | organic acid (%) | preservation conditions | wt% of granule caking | |
|---|---|---|---|---|---|
| | | | | Comparative Example without pullulan | Example pullulan addition |
| sorbitol | citric acid | 10 | 40°C×6 days | 100 | 100 |
| | malic acid | 10 | 40°C×6 days | 100 | 100 |
| maltitol | citric acid | 10 | 40°C×6 days | 21 | 16 |
| | malic acid | 10 | 40°C×6 days | 5 | 16 |
| | citric acid | 10 | 35°C×4 days | 28 | 8 |
| | malic acid | 10 | 35°C×4 days | 18 | 3 |

Table 7-2

| lactose | citric acid | 30 | 40°C×4 days | 100 | 100 |
|---|---|---|---|---|---|
| | malic acid | 30 | 40°C×4 days | 0 | 0 |
| sucrose | citric acid | 10 | 40°C×6 days | 100 | 3 |
| | malic acid | 10 | 40°C×6 days | 11 | 0 |
| glucose | citric acid | 30 | 40°C×4 days | 17 | 0 |
| | malic acid | 30 | 40°C×4 days | 100 | 0 |
| fructose | citric acid | 10 | 40°C×6 ays | 100 | 100 |
| | malic acid | 10 | 40°C×6 days | 100 | 100 |
| trehalose | citric acid | 10 | 40°C×6 days | 29 | 100 |
| | malic acid | 10 | 40°C×6 days | 33 | 13 |
| | citric acid | 10 | 40°C×4 days | 9 | 0 |
| | malic acid | 10 | 40°C×4 days | 5 | 0 |
| palatinose | citric acid | 10 | 40°C×6 days | 12 | 0 |
| | malic acid | 10 | 40°C×6 days | 33 | 6 |

Table 8

| sugar or sugar alcohol | organic acid | organic acid (%) | preservation conditions | wt% of granule caking | |
|---|---|---|---|---|---|
| | | | | Comparative Example without dextrin | Example dextrin addition |
| erythritol | citric acid | 10 | 40°C×4 days | 37 | 0 |
| | malic acid | 10 | 40°C×4 days | 33 | 0 |
| | ascorbic acid | 10 | 40°C×6 days | 100 | 35 |
| | tartaric acid | 10 | 40°C×6 days | 100 | 47 |
| | succinic acid | 10 | 40°C×6 days | 32 | 4 |

(continued)

| sugar or sugar alcohol | organic acid | organic acid (%) | preservation conditions | wt% of granule caking | |
| --- | --- | --- | --- | --- | --- |
| | | | | Comparative Example without dextrin | Example dextrin addition |
| mannitol | citric acid | 10 | 40°C×4 days | 100 | 0 |
| | malic acid | 10 | 40°C×4 days | 51 | 0 |
| | ascorbic acid | 10 | 40°C×6 days | 100 | 13 |
| | tartaric acid | 10 | 40°C×6 days | 14 | 1 |
| | succinic acid | 10 | 40°C×6 days | 100 | 60 |

(2) Fluidized bed granulation method

Comparative Example 4 (without pullulan addition)

Erythritol (finely milled powder, 800 g) and anhydrous citric acid (ground product, 100 g) were weighed (amount of organic acid added 11.1%), mixed in a PE bag with hands, and passed through a sieve with aperture of 500 $\mu$m. The sieved product was granulated in a fluidized bed granulator (POWREX CORPORATION Multiplex MP-01) while spraying 1% cornstarch solution at 6 g/min for 20 min and at 10 g/min for 38 min under the conditions of charge air temperature 70°C, air amount 70 m$^3$/hr, spray pressure 0.12 MPa. After granulation, the granules were dried for about 20 min under the conditions of charge air temperature 70°C, air amount 70 m$^3$/hr.
Xylitol (finely milled powder), sorbitol or maltitol (powder MABIT(R) 100M, HAYASHIBARA SHOJI. INC, 126 g) and anhydrous citric acid (ground product) or malic acid (14 g) were weighed (amount of organic acid added 10%), mixed in a PE bag with hands, and passed through a sieve with aperture of 710 $\mu$m. The sieved product was granulated in a fluidized bed granulator (Freund Corporation, FL-Labo) while spraying water at 1 g/min for 21 min under the conditions of charge air temperature 50°C, air amount 0.4 m$^3$/min, spray flow 25 NL/min[1]. After granulation, the granules were dried for about 3 min under the conditions of charge air temperature 50°C, air amount 0.4 m$^3$/min and passed through a sieve with aperture of 710 $\mu$m.
[1]1 NL/min = air in a standard state (pressure 0.1013 MPa, temperature 0°C, humidity 0%) flows at 1 litter per minute.

Example 5 (with pullulan addition)

Pullulan (0.5 g) was added to the preparation (4 g) of the above-mentioned Comparative Example 4, and they were mixed in a PE bag for about 5 min.

Experimental Example 2

The preparations of Comparative Example 4 and Example 5 were evaluated for the level of caking according to the preservation method and evaluation method described above.
The results are shown in Table 9. Also in the fluidized bed granulation method, pullulan, which is a water-soluble polysaccharide, showed a caking suppressive effect against caking caused by mixing erythritol, xylitol, sorbitol or maltitol and an organic acid.
Table 9

| sugar or sugar alcohol | organic acid | organic acid (%) | preservation conditions | wt% of granule caking | |
| --- | --- | --- | --- | --- | --- |
| | | | | Comparative Example 4 without pullulan | Example 5 pullulan addition |
| erythritol | citric acid | 11.1 | 40°C×7 days | 15 | 0 |
| xylitol | citric acid | 10 | 40°C×3 days | 94 | 0 |
| | malic acid | 10 | 40°C×3 days | 100 | 4 |

(continued)

| sugar or sugar alcohol | organic acid | organic acid (%) | preservation conditions | wt% of granule caking | |
|---|---|---|---|---|---|
| | | | | Comparative Example 4 without pullulan | Example 5 pullulan addition |
| maltitol | citric acid | 10 | 40°C×3 days | 37 | 7 |
| | malic acid | 10 | 40°C×3 days | 21 | 2 |
| sorbitol | citric acid | 10 | 40°C×3 days | 25 | 0 |
| | malic acid | 10 | 40°C×3 days | 9 | 0 |

(3) powder mixing method

Comparative Example 5 (without pullulan)

Xylitol (finely milled powder), sorbitol, maltitol (powder MABIT(R) 100M, HAYASHIBARA SHOJI. INC), fructose or trehalose (22.5 g) and anhydrous citric acid (ground product) or malic acid (2.5 g) were weighed as shown in Table 10 and mixed in a mortar (amount of organic acid added 10%).

Example 6 (with pullulan)

Pullulan (0.5 g) was added to the preparation (4 g) produced by the method shown in the above-mentioned Comparative Example 5, and they were mixed in a PE bag for about 5 min.
Table 10

| ingredient | amount added (g) | | | | | | | | | |
|---|---|---|---|---|---|---|---|---|---|---|
| xylitol | 22.5 | 22.5 | - | - | - | - | - | - | - | - |
| sorbitol | - | - | 22.5 | 22.5 | - | - | - | - | - | - |
| maltitol | - | - | - | - | 22.5 | 22.5 | - | - | - | - |
| fructose | - | - | - | - | - | - | 22.5 | 22.5 | - | - |
| trehalose | - | - | - | - | - | - | - | - | 22.5 | 22.5 |
| anhydrous citric acid (ground product) | 2.5 | - | 2.5 | - | 2.5 | - | 2.5 | - | 2.5 | - |
| malic acid | - | 2.5 | - | 2.5 | - | 2.5 | - | 2.5 | - | 2.5 |

Experimental Example 3

The preparations of Comparative Example 5 and Example 6 were evaluated for the level of caking according to the preservation method and evaluation method described above.
The results are shown in Table 11. It was shown that mixing of sugar or sugar alcohol and organic acid caused caking, and pullulan, which is a water-soluble polysaccharide, showed a caking suppressive effect therefor.
Table 11

| sugar or sugar alcohol | organic acid | organic acid (%) | wt% of granule caking | |
|---|---|---|---|---|
| | | | Comparative Example 5 without pullulan | Example 6 pullulan addition |
| xylitol | citric acid | 10 | 94 | 26 |
| | malic acid | 10 | 98 | 37 |

(continued)

| sugar or sugar alcohol | organic acid | organic acid (%) | wt% of granule caking | |
|---|---|---|---|---|
| | | | Comparative Example 5 without pullulan | Example 6 pullulan addition |
| sorbitol | citric acid | 10 | 75 | 6 |
| | malic acid | 10 | 36 | 2 |
| maltitol | citric acid | 10 | 41 | 15 |
| | malic acid | 10 | 50 | 16 |
| fructose | citric acid | 10 | 32 | 1 |
| | malic acid | 10 | 39 | 1 |
| trehalose | citric acid | 10 | 20 | 2 |
| | malic acid | 10 | 56 | 9 |
| preservation conditions: 40°C×3 days | | | | |

3. Comparison of caking suppressive effects of water-soluble polymer and pullulan

Erythritol (finely milled powder) or mannitol (22.5 g) and anhydrous citric acid or malic acid (2.5 g) were weighed and mixed in a mortar (amount of organic acid added 10%). They were kneaded in a mortar for about 5 min while gradually adding water (0.4 g), and further kneaded in a mortar for about 5 min while gradually adding water (0.4 g). The kneaded product was dried at 40°C for about 15 min, and passed through a sieve with aperture of 500 $\mu$m. The product was dried at 40°C for about 1 - 2 hr, and passed through a sieve with aperture of 500 $\mu$m.
The following water-soluble polymer or pullulan (0.5 g) was added to the preparation (4 g), and they were mixed in a PE bag for about 5 min.
water-soluble polymer: PVA (polyvinyl alcohol:GOHSENOL EG-05, Nippon synthesis chemical Industry Co., Ltd.),
PVP (polyvinylpyrrolidone: Kollidon CL, BASF),
CMC-Na (sodium carboxymethylcellulose: Nacalai Tesque),
CVP (carboxyvinyl polymer: CARBOPOL 934P NF, BF Goodrich)

Experimental Example 4

The preparations produced above were evaluated for the level of caking according to the preservation method and evaluation method described above.
The results are shown in Table 12. By comparison of the caking suppressive effects of the above-mentioned polymer and pullulan, the caking suppressive effect of pullulan was markedly higher.
Table 12

| | | wt% of granule caking | | | | |
|---|---|---|---|---|---|---|
| | | Example pullulan | Comp. Ex. PVP | Comp. Ex. PVA | Comp. Ex. CMC-Na | Comp. Ex. CVP |
| mannitol | citric acid | 0 | 100 | 31 | 68 | 98 |
| | malic acid | 2 | 59 | 29 | 9 | 93 |
| erythritol | citric acid | 0 | 100 | 8 | 8 | 100 |
| | malic acid | 0 | 100 | 29 | 58 | 100 |

preservation conditions: 40°C×4 days

4. Comparison of caking suppressive effects of pullulan and/or dextrin and other water-soluble polysaccharides

Erythritol (finely milled powder) or mannitol (22.5 g) and anhydrous citric acid (ground product) or malic acid (2.5 g) were weighed and mixed in a mortar. They were kneaded in a mortar for about 5 min while gradually adding water (0.4 g), and further kneaded in a mortar for about 5 min while gradually adding water (0.4 g). The kneaded product was dried at 40°C for about 15 min, and passed through a sieve with aperture of 500 $\mu$m. The product was dried at 40°C for about 1 - 2 hr, and passed through a sieve with aperture of 500 $\mu$m.
Pullulan, dextrin or water-soluble polysaccharide (0.5 g) shown in Table 13 was added to the preparation (4 g), and they were mixed in a PE bag for about 5 min.

Experimental Example 5

The preparations produced above were evaluated for the level of caking according to the preservation method and evaluation method described above.
The results are shown in Table 13. As compared to other water-soluble polysaccharides, pullulan and dextrin showed a markedly higher caking suppressive effect. It was shown that pullulan and dextrin are extremely superior caking suppressing agents.
Table 13

| | wt% of granule caking | | | |
| | mannitol | | erythritol | |
| | citric acid | malic acid | citric acid | malic acid |
|---|---|---|---|---|
| Comparative Example none | 100 | 51 | 37 | 33 |
| Example pullulan | 0 | 2 | 0 | 0 |
| Example dextrin | 0 | 2 | 0 | 0 |
| Comparative Example carageenan | 94 | 50 | 100 | 100 |
| Comparative Example xanthan gum | 90 | 100 | 100 | 100 |
| Comparative Example pectin | 83 | 49 | 36 | 40 |
| Comparative Example gum arabic powder | 100 | 84 | 100 | 100 |
| Comparative Example alginic acid Na | 94 | 35 | 54 | 46 |

preservation conditions   40°C×4 days

5. Consideration of mixing ratio of organic acid and water-soluble polysaccharides (active ingredient: blonanserin)

(1) Consideration of amount ratio active ingredient: blonanserin
sugar alcohol: erythritol (finely milled powder) or mannitol organic acid: anhydrous citric acid (ground product) or malic acid
water-soluble polysaccharides: pullulan

Comparative Example 6 (without pullulan addition)

Blonanserin (0.2 g, fixed), sugar alcohol and organic acid (ground product) were weighed as shown in Table 14, and a preparation was produced according to the above-mentioned extrusion granulation method.
Table 14

| | | amount of organic acid added 1) | | | |
|---|---|---|---|---|---|
| | | 0.2% | 5% | 10% | 30% |
| amount of pullulan added 2) | 0% | 24.8/0.05 | 23.6/1.2 | 22.3/2.5 | 17.4/7.4 |
| | 0.1% | 24.8/0.05 | 23.6/1.2 | — | — |
| | 1% | 24.8/0.05 | 23.6/1.2 | 22.3/2.5 | — |
| | 3% | — | 23.6/1.2 | 22.3/2.5 | — |
| | 5% | — | — | — | 17.4/7.4 |
| | 10% | — | 23.6/1.2 | 22.3/2.5 | — |
| | 20% | — | — | — | 17.4/7.4 |

1) Expressed by value obtained by dividing weight of organic acid by total weight of preparation excluding caking suppressing agent, and multiplying the value by 100.
2) Expressed by value obtained by dividing weight of pullulan by total weight of preparation excluding caking suppressing agent, and multiplying the value by 100.
Number in column shows amount (g) of "sugar alcohol/organic acid" added to the formulation.

Example 7 (with pullulan addition)

Pullulan in an amount shown in Table 15 was added to the preparation (4 g) of the above-mentioned Comparative Example, and they were mixed in a PE bag for about 5 min.
Table 15

| | | amount of organic acid added 1) | | | |
|---|---|---|---|---|---|
| | | 0.2% | 5% | 10% | 30% |
| amount of pullulan added 2) | 0% | 0 | 0 | 0 | 0 |
| | 0.1% | 0.004 | 0.004 | — | — |
| | 1% | 0.04 | 0.04 | 0.04 | — |
| | 3% | — | 0.12 | 0.12 | — |
| | 5% | — | — | — | 0.2 |
| | 10% | — | 0.4 | 0.4 | — |
| | 20% | — | — | — | 0.8 |

1) Expressed by value obtained by dividing weight of organic acid by total weight of preparation excluding caking suppressing agent, and multiplying the value by 100.
2) Expressed by value obtained by dividing weight of pullulan by total weight of preparation excluding caking suppressing agent, and multiplying the value by 100.
Number in column shows amount (g) of pullulan added to the formulation.

Experimental Example 6

The preparations of Comparative Example 6 and Example 7 were preserved at 40°C for 4 days and evaluated according to the evaluation method described above.

(2) Test results

1) Blonanserin, mannitol, citric acid formulation

The results are shown in Table 16. In the case of mannitol, pullulan at 0.1% could suppress caking caused by citric acid (0.2% and 5%) as compared to no addition of pullulan. Pullulan at 10% and 20% could suppress caking caused by citric acid (10% and 30%, respectively).
Table 16

| wt% of granule caking | | amount of organic acid added 1) | | | |
|---|---|---|---|---|---|
| | | 0.2% | 5% | 10% | 30% |
| amount of pullulan added 2) | Comparative Example 0% | 18 | 100 | 100 | 100 |
| | Example 0.1% | 4 | 42 | - | - |
| | Example 1% | 0 | 38 | 100 | - |
| | Example 3% | - | 19 | 100 | - |
| | Example 5% | - | - | - | 100 |
| | Example 10% | - | 0 | 12 | - |
| | Example 20% | - | - | - | 1 |
| 1) Expressed by value obtained by dividing weight of organic acid by total weight of preparation excluding caking suppressing agent, and multiplying the value by 100. 2) Expressed by value obtained by dividing weight of pullulan by total weight of preparation excluding caking suppressing agent, and multiplying the value by 100. | | | | | |

2) Blonanserin, mannitol, malic acid formulation

The results are shown in Table 17. In the case of mannitol, pullulan at 0.1% could suppress caking caused by malic acid (0.2%) as compared to no addition of pullulan. Pullulan could suppress caking caused by malic acid (5%) in a dose-dependent manner, and pullulan at 1% and 5% could suppress caking caused by citric acid (10% and 30%, respectively).
Table 17

| wt% of granule caking | | amount of organic acid added 1) | | | |
|---|---|---|---|---|---|
| | | 0.2% | 5% | 10% | 30% |
| amount of pullulan added 2) | Comparative Example 0% | 100 | 100 | 100 | 91 |
| | Example 0.1% | 47 | 88 | - | - |
| | Example 1% | 0 | 87 | 40 | - |
| | Example 3% | - | 58 | 23 | - |
| | Example 5% | - | - | - | 19 |
| | Example 10% | - | 0 | 0 | - |
| | Example 20% | - | - | - | 0 |
| 1) Expressed by value obtained by dividing weight of organic acid by total weight of preparation excluding caking suppressing agent, and multiplying the value by 100.<br>2) Expressed by value obtained by dividing weight of pullulan by total weight of preparation excluding caking suppressing agent, and multiplying the value by 100. | | | | | |

3) Blonanserin, erythritol, citric acid formulation

The results are shown in Table 18. In the case of erythritol, caking did not occur with organic acid at 0.2%. Pullulan could suppress caking caused by citric acid at 5% in a dose-dependent manner, as compared to no addition of pullulan, and pullulan at 10% could almost completely suppress caking. Pullulan at 1% and 5% could suppress caking caused by citric acid (10% and 30%, respectively).
Table 18

| wt% of granule caking | | amount of organic acid added 1) | | | |
|---|---|---|---|---|---|
| | | 0.2% | 5% | 10% | 30% |
| amount of pullulan added 2) | Comparative Example 0% | 0 | 100 | 88 | 100 |
| | Example 0.1% | 0 | 93 | - | - |
| | Example 1% | 0 | 76 | 38 | - |
| | Example 3% | - | 52 | 33 | - |
| | Example 5% | - | - | - | 0 |
| | Example 10% | - | 1 | 0 | - |
| | Example 20% | - | - | - | 0 |
| 1) Expressed by value obtained by dividing weight of organic acid by total weight of preparation excluding caking suppressing agent, and multiplying the value by 100.<br>2) Expressed by value obtained by dividing weight of pullulan by total weight of preparation excluding caking suppressing agent, and multiplying the value by 100. | | | | | |

4) Blonanserin, erythritol, malic acid formulation

The results are shown in Table 19. In the case of erythritol, pullulan at 0.1% could suppress caking caused by malic acid at 0.2%, as compared to no addition of pullulan. Pullulan could suppress caking caused by malic acid at 5% and 10% in a dose-dependent manner, and pullulan at 5% could suppress caking caused by malic acid at 30%.
Table 19

| wt% of granule caking | | amount of organic acid added 1) | | | |
|---|---|---|---|---|---|
| | | 0.2% | 5% | 10% | 30% |
| amount of pullulan added 2) | Comparative Example 0% | 100 | 96 | 100 | 100 |
| | Example 0.1% | 49 | 72 | - | - |
| | Example 1% | 0 | 51 | 93 | - |
| | Example 3% | - | 37 | 83 | - |
| | Example 5% | - | - | - | 30 |
| | Example 10% | - | 0 | 1 | - |
| | Example 20% | - | - | - | 0 |
| 1) Expressed by value obtained by dividing weight of organic acid by total weight of preparation excluding caking suppressing agent, and multiplying the value by 100. 2) Expressed by value obtained by dividing weight of pullulan by total weight of preparation excluding caking suppressing agent, and multiplying the value by 100. | | | | | |

6. Results of consideration of various active ingredients active ingredients: shown in Table 20.

sugar alcohol: erythritol (finely milled powder) or mannitol organic acid: anhydrous citric acid (ground product) or malic acid

Comparative Example 7 (without addition of pullulan and dextrin)

Various active ingredients, sugar alcohol and organic acid were weighed as shown in Table 20 and a preparation was produced according to the method described in the above-mentioned extrusion granulation method or powder mixing method.

In the case of fluidized bed granulation method, blonanserin (1.1 g), mannitol or erythritol (finely milled powder, 125.0 g), and anhydrous citric acid (ground product) or malic acid (13.9 g) were measured and mixed in a PE bag with hands, and passed through a sieve with aperture of 710 $\mu$m. The sieved product was granulated in a fluidized bed granulator (Freund Corporation, FL-Labo) while spraying water at 1 g/min for 21 min under the conditions of charge air temperature 50°C, air amount 0.4 m$^3$/min, spray flow 25 NL/min. After granulation, the granules were dried for about 3 min under the conditions of charge air temperature 50°C, air amount 0.4 m$^3$/min and passed through a sieve with aperture of 710 $\mu$m.

Example 8 (with addition of pullulan)

Pullulan (0.5 g) was added to the preparation (4 g) of the above-mentioned Comparative Example 7, and they were mixed in a PE bag for about 5 min. The particle size (90% diameter) of the preparation after pullulan addition was 285 $\mu$m.

Example 9 (with addition of dextrin)

Dextrin (0.5 g) was added to the preparation (4 g) of the above-mentioned Comparative Example 7, and they were mixed in a PE bag for about 5 min.

Experimental Example 7

The preparations of Comparative Example 7, Example 8 and Example 9 were preserved at 40°C for 4 days and evaluated according to the evaluation method described above.
Table 20

| active ingredient | | amount of sugar alcohol added (g) | amount of organic acid added (g) | granulation method | water-soluble polysaccharides | |
|---|---|---|---|---|---|---|
| ingredient name | amount added (g) | | | | Comparative Example | Example |
| zonisamide | 2.5 | erythritol 20.3 | anhydrous citric acid 2.25 | extrusion | none | pullulan |
| blonanserin | 0.2 | mannitol 22.3 | malic acid 2.48 | powder mixing | none | pullulan dextrin |
| blonanserin | 0.2 | erythritol 22.3 | anhydrous citric acid 2.48 | powder mixing | none | pullulan |

The results of pullulan addition are shown in Table 21, and the results of dextrin addition are shown in Table 22. A caking suppressive effect of pullulan and dextrin was observed in every active ingredient.
Table 21

| active ingredient | sugar alcohol | organic acid | granulation method | wt% of granule caking | |
|---|---|---|---|---|---|
| | | | | Comparative Example 7 without pullulan | Example 9 pullulan addition |
| zonisamide | erythritol | citric acid | extrusion | 21 | 0 |
| blonanserin | mannitol | malic acid | powder mixing | 100 | 11 |
| blonanserin | erythritol | citric acid | powder mixing | 49 | 0 |
| blonanserin | mannitol | malic acid | fluidized-bed | 66 | 0 |
| blonanserin | erythritol | citric acid | fluidized-bed | 100 | 0 |
| preservation conditions: extrusion, powder mixing 40°C×4 days, fluidized-bed 40°C×3 days | | | | | |

Table 22

| active ingredient | sugar alcohol | organic acid | granulation method | wt% of granule caking | |
|---|---|---|---|---|---|
| | | | | Comparative Example 7 without dextrin | Example 9 dextrin addition |
| blonanserin | mannitol | malic acid | powder mixing | 100 | 11 |
| blonanserin | mannitol | malic acid | fluidized-bed | 66 | 0 |
| blonanserin | erythritol | citric acid | fluidized-bed | 100 | 0 |
| preservation conditions: powder mixing 40°C×4 days, fluidized-bed 40°C×3 days | | | | | |

active ingredients: shown in Table 23.
sugar alcohol: erythritol (finely milled powder)
organic acid: anhydrous citric acid (ground product), malic acid

Comparative Example 8 (without addition of pullulan and dextrin)

The active ingredient, erythritol (finely milled powder) and an organic acid were weighed as shown in Table 23 and Table 25, and a preparation was produced according to the method described in the above-mentioned extrusion granulation method.

Example 10 (with addition of pullulan)

Pullulan (0.5 g) was added to the preparation (4 g) of the above-mentioned Comparative Example 8, and they were mixed in a PE bag for about 5 min.

Example 11 (with addition of dextrin)

Dextrin (0.5 g) was added to the preparation (4 g) of the above-mentioned Comparative Example 8, and they were mixed in a PE bag for about 5 min.

Experimental Example 8

The preparations of Comparative Example 8, Example 10 and Example 11 were preserved at 40°C for 4 days and evaluated according to the evaluation method described above.
Table 23

| active ingredient | | amount of sugar alcohol added (g) | amount of organic acid added (g) | granulation method | water-soluble polysaccharides | |
| --- | --- | --- | --- | --- | --- | --- |
| ingredient name | amount added (g) | | | | Comparative Example | Example |
| perospirone hydrochloride hydrate | 0.43 | erythritol 22.1 | anhydrous citric acid 2.5 | extrusion | none | pullulan |
| gliclazide | 1.0 | erythritol 21.5 | anhydrous citric acid 2.5 | extrusion | none | pullulan |
| lurasidone hydrochloride | 2.0 | erythritol 20.7 | anhydrous citric acid 2.30 | extrusion | none | pullulan |
| compound (I) | 0.5 | erythritol 22.1 | malic acid 2.45 | extrusion | none | pullulan |
| compound (I) | 0.5 | erythritol 22.1 | anhydrous citric acid 2.45 | extrusion | none | pullulan |
| compound (II) | 1.3 | erythritol 22.5 | anhydrous citric acid 1.25 | extrusion | none | pullulan |
| compound (III) | 0.3 | erythritol 22.3 | anhydrous citric acid 2.5 | extrusion | none | pullulan |

The results are shown in Table 24. A caking suppressive effect of pullulan was observed for every active ingredient.
Table 24

| active ingredient | sugar alcohol | organic acid | granulation method | weight (g) of granule caking | |
|---|---|---|---|---|---|
| | | | | Comparative Example without pullulan | Example pullulan addition |
| perospirone hydrochloride hydrate | erythritol | citric acid | extrusion | 97 | 0 |
| gliclazide | erythritol | citric acid | extrusion | 60 | 0 |
| lurasidone hydrochloride | erythritol | citric acid | extrusion | 100 | 0 |
| compound (I) | erythritol | malic acid | extrusion | 76 | 0 |
| compound (I) | erythritol | citric acid | extrusion | 100 | 0 |
| compound (II) | erythritol | citric acid | extrusion | 100 | 30 |
| compound (III) | erythritol | citric acid | extrusion | 100 | 0 |
| preservation conditions: 40°C×4 days | | | | | |

Table 25

| active ingredient | | amount of sugar alcohol added (g) | amount of organic acid added (g) | granulation method | water-soluble polysaccharides | |
|---|---|---|---|---|---|---|
| ingredient name | amount added (g) | | | | Comparative Example | Example |
| compound (I) | 0.5 | erythritol 22.1 | malic acid 2.45 | extrusion | none | dextrin |

The results are shown in Table 26. A caking suppressive effect of dextrin was observed for compound (I).
Table 26

| active ingredient | sugar alcohol | organic acid | granulation method | weight (g) of granule caking | |
|---|---|---|---|---|---|
| | | | | Comparative Example without dextrin | Example dextrin addition |
| compound (I) | erythritol | malic acid | extrusion | 76 | 0 |
| preservation conditions: 40°C×4 days | | | | | |

Example 12

Mosapride citrate dihydrate (0.13 g), mannitol (22.4 g) and malic acid (2.49 g) were weighed and a preparation was produced according to the method described in the above-mentioned extrusion granulation method (amount of organic acid added 10%). Pullulan (0.5 g) was added to the preparation (4 g), and they were mixed in a PE bag for about 5 min, preserved at 40°C for 4 days and evaluated according to the evaluation method described above.
As a result, wt% of granule caking was 0%.

Example 13

Mosapride citrate dihydrate (0.13 g), erythritol (finely milled powder, 22.4 g) and anhydrous citric acid (2.5 g) were weighed and a preparation was produced according to the method described in the above-mentioned extrusion granulation method (amount of organic acid added 10%). Pullulan (0.5 g) was added to the preparation (4 g), and they were mixed in a PE bag for about 5 min, preserved at 40°C for 4 days and evaluated according to the evaluation method described above.

As a result, wt% of granule caking was 0%.

Production of active ingredient-containing particles

Acetaminophen was coated to a coating amount of 10% to give acetaminophen-containing particles. The coating film component used contained aquacoat (Asahikasei Chemical Industry), triacetine and mannitol at 100:25:50 wt%.

Consideration using acetaminophen-containing particles

Comparative Example 9 (without addition of pullulan)

Erythritol (finely milled powder, 22.2 g) and anhydrous citric acid (pulverized product, 2.5 g) were weighed and a preparation was produced according to the method described in the above-mentioned extrusion granulation method. The obtained granulation product (3.95 g) was mixed with acetaminophen-containing particles (0.0528 g) in this ratio to give a preparation (Table 27).

Example 14 (with addition of pullulan)

Pullulan (0.5 g) was added to the preparation (4 g) of the above-mentioned Comparative Example 9, and they were mixed in a PE bag for about 5 min.

Experimental Example 9

The preparations of Comparative Example 9 and Example 14 were preserved at 40°C for 4 days and evaluated according to the evaluation method described above.
Table 27

| active ingredient | | amount of sugar alcohol added (g) | amount of organic acid added (g) | granulation method | water-soluble polysaccharides | |
|---|---|---|---|---|---|---|
| ingredient name | amount added (g) | | | | Comp. Ex. | Ex. |
| acetaminophen-containing particles | 0.33 | erythritol 22.2 | anhydrous citric acid 2.5 | extrusion | none | pullulan |

The results are shown in Table 28. Pullulan showed a caking suppressive effect even for particles containing an active ingredient coated with a coating agent, such as acetaminophen-containing particles.
Table 28

| active ingredient | sugar alcohol | organic acid | granulation method | weight (g) of granule caking | |
|---|---|---|---|---|---|
| | | | | Comparative Example without pullulan | Example pullulan addition |
| acetaminophen-containing particles | erythritol | citric acid | extrusion | 100 | 0 |
| preservation conditions: 40°C×4 days | | | | | |

Long-term preservation stability at room temperature

Example 15

Mosapride citrate dihydrate (0.13 g), mannitol (22.4 g) and malic acid (2.49 g) were weighed and a preparation was produced according to the method described in the above-mentioned extrusion granulation method. Pullulan (0.5 g) was added to the preparation (4 g), and they were mixed in a PE bag for about 5 min.

Zonisamide (2.5 g), erythritol (20.3 g) and anhydrous citric acid (2.25 g) were weighed and a preparation was produced according to the method described in the above-mentioned extrusion granulation method. Pullulan (0.5 g) was added to the preparation (4 g), and they were mixed in a PE bag for about 5 min.

Blonanserin (0.2 g), mannitol (22.3 g) and malic acid (2.5 g) were weighed and a preparation was produced according to the method described in the above-mentioned extrusion granulation method. Pullulan (0.5 g) was added to the preparation (4 g), and they were mixed in a PE bag for about 5 min.

Blonanserin (0.2 g), mannitol (22.3 g) and anhydrous citric acid (2.5 g) were weighed and a preparation was produced according to the method described in the above-mentioned extrusion granulation method. Pullulan (0.5 g) was added to the preparation (4 g), and they were mixed in a PE bag for about 5 min.

Blonanserin (0.2 g), erythritol (22.3 g) and malic acid (2.5 g) were weighed and a preparation was produced according to the method described in the above-mentioned extrusion granulation method. Pullulan (0.5 g) was added to the preparation (4 g), and they were mixed in a PE bag for about 5 min.

Blonanserin (0.2 g), erythritol (22.3 g) and anhydrous citric acid (2.5 g) were weighed and a preparation was produced according to the method described in the above-mentioned extrusion granulation method. Pullulan (0.5 g) was added to the preparation (4 g), and they were mixed in a PE bag for about 5 min.

The granules obtained above were preserved at room temperature (20 - 30°C) for 7 months, and evaluated according to the evaluation method described above. As a result, wt% of granule caking was 0% under any combinations.

Long-term preservation stability at room temperature

Comparative Example 10

Blonanserin (1.1 g), erythritol (125.0 g) and anhydrous citric acid (13.9 g) were weighed and a preparation was produced according to the method described in the fluidized bed granulation method described in Comparative Example 7. Pullulan (0.5 g) was added to the preparation (4 g), and they were mixed in a PE bag for about 5 min.

Blonanserin (1.1 g), mannitol (125.0 g) and malic acid (13.9 g) were weighed and a preparation was produced according to the method described in the fluidized bed granulation method described in Comparative Example 7. Pullulan (0.5 g) was added to the preparation (4 g), and they were mixed in a PE bag for about 5 min.

Example 16

Pullulan (0.5 g) was added to the preparation (4 g) of the above-mentioned Comparative Example 10, and they were mixed in a PE bag for about 5 min.

The preparations of Comparative Example 10 and Example 16 were preserved at room temperature (20 - 30°C) for 7 months and evaluated according to the evaluation method described above. The results are shown in Table 29. Pullulan showed a caking suppressive effect for both combinations after preservation at room temperature.

Table 29

| active ingredient | sugar alcohol | organic acid | granulation method | weight (g) of granule caking | |
|---|---|---|---|---|---|
| | | | | Comparative Example without pullulan | Example pullulan addition |
| blonanserin | erythritol | citric acid | fluidized-bed | 61 | 0 |
| blonanserin | mannitol | malic acid | fluidized-bed | 43 | 0 |
| preservation conditions: room temperature×7 months | | | | | |

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2006-290561 filed in Japan, the contents of which are incorporated in full herein by this reference.

## Claims

1. A granular preparation with suppressed caking, which comprises an active ingredient other than biguanide, a sugar or a sugar alcohol, an organic acid, and at least one water-soluble polysaccharide selected from the group consisting of pullulan and dextrin.

2. The granular preparation of claim 1, wherein the sugar or sugar alcohol is at least one selected from the group consisting of erythritol, mannitol, xylitol, sorbitol, maltitol and trehalose.

3. The granular preparation of claim 1, wherein the sugar or sugar alcohol is at least one selected from the group consisting of erythritol and mannitol.

4. The granular preparation of any one of claims 1 to 3, wherein the organic acid is at least one selected from the group consisting of citric acid, malic acid, ascorbic acid, tartaric acid, succinic acid and hydrates thereof.

5. The granular preparation of any one of claims 1 to 3, wherein the organic acid is at least one selected from the group consisting of citric acid, malic acid and hydrates thereof.

6. The granular preparation of any one of claims 1 to 5, wherein the water-soluble polysaccharide is pullulan.

7. The granular preparation of any one of claims 1 to 6, wherein the proportion of sugar or sugar alcohol is 20 - 99.8 wt% of the whole preparation.

8. The granular preparation of any one of claims 1 to 7, wherein the proportion of the organic acid is 0.1 - 30 wt% of the whole preparation.

9. The granular preparation of any one of claims 1 to 8, wherein the proportion of the water-soluble polysaccharide is 0.1 - 20 wt% of the whole preparation.

10. The granular preparation of any one of claims 1 to 9, wherein the weight ratio of an addition amount of the water-soluble polysaccharide to an addition amount of the organic acid is 0.01 - 100.

11. The granular preparation of any one of claims 1 to 10, which has a 90% diameter of not more than 1700 $\mu$m.

12. The granular preparation of any one of claims 1 to 11, wherein the dosage form is an orally-disintegrating preparation.

13. A method of suppressing caking of a granular preparation, which comprises a step of adding at least one water-soluble polysaccharide selected from the group consisting of pullulan and dextrin to a preparation comprising an active ingredient other than biguanide, a sugar or a sugar alcohol and an organic acid.

14. A method of producing a granular preparation with suppressed caking, which comprises a step of adding at least one water-soluble polysaccharide selected from the group consisting of pullulan and dextrin to an active ingredient other than biguanide, a sugar or sugar alcohol, and an organic acid.

15. A caking suppressing agent comprising at least one water-soluble polysaccharide selected from the group consisting of pullulan and dextrin, which agent is used for a granular preparation comprising an active ingredient other than biguanide, a sugar or a sugar alcohol and an organic acid.

16. A granular preparation with suppressed caking, which comprises a sugar or a sugar alcohol, an organic acid, and at least one water-soluble polysaccharide selected from the group consisting of pullulan and dextrin, and does not comprise biguanide.

## INTERNATIONAL SEARCH REPORT

| International application No. |
| --- |
| PCT/JP2007/070854 |

A. CLASSIFICATION OF SUBJECT MATTER
*A61K9/14*(2006.01)i, *A61K47/10*(2006.01)i, *A61K47/12*(2006.01)i, *A61K47/26*
(2006.01)i, *A61K47/36*(2006.01)i

According to International Patent Classification (IPC) or to both national classification and IPC

B. FIELDS SEARCHED

Minimum documentation searched (classification system followed by classification symbols)
A61K9/14, A61K47/10, A61K47/12, A61K47/26, A61K47/36

Documentation searched other than minimum documentation to the extent that such documents are included in the fields searched
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| P,X | WO 2006/118137 A1 (DAINIPPON SUMITOMO PHARMA CO., LTD.), 09 November, 2006 (09.11.06), Full text; particularly, Claims 1 to 15 (Family: none) | 1-16 |
| X | JP 2005-314413 A (ONO PHARM CO., LTD.), 10 November, 2005 (10.11.05), Full text; particularly, examples 1 to 4, 6, 11 to 14; Par. Nos. [0016], [0017], [0024], [0026] (Family: none) | 1-16 |
| X | JP 2002-524535 A (INHALE THERAPEUTIC SYSTEMS), 06 August, 2002 (06.08.02), Full text; particularly, Claims 1, 2; Formulation D of Example 4 & WO 2000/15262 A1      & EP 1117442 A1 | 1-5,7-11, 13-16 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 10 December, 2007 (10.12.07) | Date of mailing of the international search report 18 December, 2007 (18.12.07) |
| --- | --- |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2007/070854

C (Continuation).   DOCUMENTS CONSIDERED TO BE RELEVANT

| Category* | Citation of document, with indication, where appropriate, of the relevant passages | Relevant to claim No. |
|---|---|---|
| X | JP 07-157431 A  (TOYO YAKUHIN KOGYO KABUSHIKI KAISHA),<br>20 June, 1995 (20.06.95),<br>Full text; particularly, example 1<br>(Family: none) | 1,4,5,7,<br>9-11,13-16 |
| A | JP 11-116464 A  (SS PHARM CO., LTD.),<br>27 April, 1999 (27.04.99),<br>Full text<br>& WO 99/18936 A1          & JP 11-116465 A<br>& JP 11-116466 A          & EP 1022021 A1<br>& US 6455053 B1 | 1-16 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9316006 A
- JP 8012582 A
- JP 2516147 B
- WO 2006118268 A
- WO 9903857 A
- JP 2006290561 A